# NEUE EUROPÄISCHE PATENTSCHRIFT

(11) **EP 0 579 091 B2**
(45) Veröffentlichungstag und Bekanntmachung des Hinweises auf die Entscheidung über den Einspruch: **26.01.2000**
(45) Hinweis auf die Patenterteilung: 24.01.1996
(21) Anmeldenummer: 93110828.6
(22) Anmeldetag: 07.07.1993
(51) Int. Cl.: C09C 1/00, C09D 7/12, C09D 11/00, C08K 3/00, C03C 4/02, C04B 33/14, A61K 7/00

(54) **Glanzpigmente mit metallsulfidhaltiger Beschichtung**
Lustrons pigments with metal sulfide coating
Pigments lustrés avec un revêtement contenant du sulfure métallique

(30) Priorität: 16.07.1992 DE 4223383
(43) Veröffentlichungstag der Anmeldung: 19.01.1994
(73) Patentinhaber: BASF Aktiengesellschaft, 67063 Ludwigshafen (DE)
(72) Erfinder: Schmid, Raimund, Dr., W-6730 Neustadt (DE); Mronga, Norbert, Dr., W-6915 Dossenheim (DE)

(56) Entgegenhaltungen:
- EP-A- 0 360 740
- EP-A- 0 362 129
- DE-A- 1 467 466
- FR-A- 2 134 538
- GB-A- 1 160 173
- US-A- 2 995 459
- US-A- 3 123 490
- XIX. Fatipec Kongress, Aachen 1988, S. 103-114
- Gmelins Handbuch der anorganischen Chemie, Wolfram, S. 184-186, Verlag Chemie Berlin, 1933
- Comprehensive Inorganic Chemistry, Vol. A, 1973, S. 1002

## Beschreibung

Die vorliegende Erfindung betrifft neue Glanzpigmente auf Basis beschichteter, plättchenförmiger, silikatischer oder metallischer Substrate, die mit
A) einer ersten Schicht aus Metalloxid,
B) einer zweiten Schicht, die ein nichtselektiv absorbierendes Metallsulfid enthält und eine Schichtdicke von 0,1 bis 50 nm aufweist, und
C) gewünschtenfalls einer dritten Schicht aus Metalloxid
   belegt sind.

Weiterhin betrifft die Erfindung die Herstellung dieser Glanzpigmente sowie ihre Verwendung zum Einfärben von Lacken, Druckfarben, Kunststoffen, Gläsern, keramischen Produkten und Zubereitungen der dekorativen Kosmetik.

Glanz- oder Effektpigmente werden in zunehmendem Maße in vielen Bereichen der Technik eingesetzt, beispielsweise in Automobillacken, in der dekorativen Beschichtung, der Kunststoffeinfärbung, in Druck-, Anstrich-, insbesondere Sicherheitsfarben sowie in der Kosmetik.

Ihre optische Wirkung beruht auf der gerichteten Reflexion an überwiegend flächig ausgebildeten, ausgerichteten metallischen oder stark lichtbrechenden Pigmentteilchen. Je nach Art der Pigmentteilchen spricht man auch von Metalleffektpigmenten (z.B. Aluminium, Zink, Kupfer oder deren Legierungen) oder Perlglanzpigmenten (z.B. auf Basis titandioxidbeschichteter Glimmer wie Muskovit, Phlogopit und Biotit, Talkum oder Glas).

Die Glanzpigmente können durch Beschichtung der Ausgangssubstrate mit dünnen Filmen aus hochbrechenden Oxiden wie Chrom(III)oxid, vor allem Eisenoxid und Titanoxid mehrphasig aufgebaut sein. Durch Interferenz und gegebenenfalls Absorption ergibt sich in diesen Fällen in Abhängigkeit von der Dicke der Oxidschicht eine Vielzahl von Farbtonvariationen; man nennt diese Pigmente auch Interferenzpigmente und bei metallischem Substrat auch Interferenzreflexionspigmente.

Durch die gerichtete Reflexion des einfallenden Lichtes an den plättchenförmigen Pigmentteilchen zeigen die z.B. in Lack ausgerichteten, beschichteten Glanzpigmente Goniochromatizität, d.h. der Farbeindruck (Helligkeit und/oder Farbton und/oder Farbsättigung) ihrer Lackierung ändert sich in Abhängigkeit vom Belichtungs- bzw. Betrachtungswinkel. Diese Effekte lassen sich auf ein kompliziertes Zusammenspiel von Reflexion und Transmission des auftreffenden Lichts zurückführen, wobei dessen Farbe durch an den Pigmentteilchen hervorgerufene Phänomene wie Interferenz an dünnen Schichten und Absorption an farbigen Zentren verändert werden kann.

Aus der US-A-2 995 459 ist die Herstellung von Glanzpigmenten durch Beschichtung der teuren, plättchenförmigen Materialien Fischsilber (Guanin), Bleicarbonat und Wismutoxychlorid mit Cadmium-, Zinn-, Eisen-, Mangan- oder Antimonsulfid bekannt. In der US-A-3 123 490 wird die Verwendung von Zinksulfid zur Herstellung von Glanzpigmenten beschrieben, die sich jedoch im Aufbau deutlich von den erfindungsgemäßen Pigmenten unterscheiden.

Plättchenförmig kristallisierende Metallsulfide wie das hochbrechende und hochreflektierende Molybdänsulfid (MoS₂) können auch direkt als einphasige Glanzpigmente verwendet werden. Glanzeffekte lassen sich zwar mit diesen Pigmenten erzeugen, jedoch ist man ohne den zusätzlichen Einsatz von weiteren Farbmitteln wie Perlglanzpigmenten oder organischen Pigmenten auf dunkelblaue oder schwarze Farbtöne beschränkt (US-A-5 063 258 oder EP-A-360 740).

Der Erfindung lag daher die Aufgabe zugrunde, neue Glanzpigmente bereitzustellen, welche die genannten Nachteile nicht aufweisen und sich insgesamt durch vorteilhafte Anwendungseigenschaften auszeichnen.

Demgemäß wurden die Glanzpigmente auf Basis beschichteter, plättchenförmiger, silikatischer oder metallischer Substrate gefunden, die mit
A) einer ersten Schicht aus Metalloxid,
B) einer zweiten Schicht, die ein nichtselektiv absorbierendes Metallsulfid enthält und eine Schichtdicke von 0,1 bis 50 nm aufweist, und C) gewünschtenfalls einer dritten Schicht aus Metalloxid
   belegt sind.

Weiterhin wurde ein Verfahren zur Herstellung von Glanzpigmenten auf Basis beschichteter, plättchenförmiger, silikatischer oder metallischer substrate, die
A) gewünschtenfalls mit einer ersten Schicht aus Metalloxid,
B) mit einer zweiten Schicht, die ein nichtselektiv absorbierendes Metallsulfid enthält, und
C) gewünschtenfalls mit einer Schicht aus Metalloxid
   belegt sind, gefunden, welches dadurch gekennzeichnet ist, daß man das gewünschtenfalls bereits mit einer Metalloxidschicht belegte Substrat
   a) zunächst in bekannter Weise durch Gasphasenzersetzung flüchtiger Metallverbindungen in Gegenwart eines Inertgases oder von Sauerstoff und/oder Wasserdampf mit der entsprechenden Metall- oder Metalloxidschicht belegt und diese dann durch Umsetzung mit einer flüchtigen, schwefelhaltigen Verbindung oder Schwefeldampf in die gewünschte metallsulfidhaltige Schicht überführt oder
   b) durch Gasphasenzersetzung flüchtiger Metallverbindungen in Gegenwart einer flüchtigen, schwefelhaltigen Verbindung oder Schwefeldampf direkt mit der metallsulfidhaltigen Schicht belegt
      sowie das so beschichtete Substrat gewünschtenfalls anschließend in üblicher Weise mit einer weiteren Metalloxidschicht belegt.

Außerdem wurde die Verwendung der Glanzpigmente zur Einfärbung von Lacken, Druckfarben, Kunststoffen, Gläsern, keramischen Produkten und Zubereitungen der dekorativen Kosmetik gefunden.

Für die erfindungsgemäßen Glanzpigmente kommen als Substrate insbesondere silikatische oder metallische Plättchen oder deren Mischungen in Betracht.

Besonders bevorzugt sind helle bzw. weiße Glimmer, insbesondere Schuppen von naß vermahlenem Muskovit. Selbstverständlich sind auch andere natürliche Glimmer wie Phlogopit, Biotit, künstliche Glimmer, Talk- und Glasschuppen geeignet.

Als metallische Substrate kommen für die erfindungsgemäßen Pigmente alle für Metalleffektpigmente bekannten Metalle in Plättchenform in Frage; z.B. sind neben Kupfer und seinen Legierungen wie Messing oder Bronzen vor allem Aluminium und seine Legierungen wie Aluminiumbronze geeignet. Bevorzugt sind Aluminiumplättchen, die in einfacher Weise durch Herausstanzen aus Aluminiumfolie oder nach üblichen Verdüsungs- oder Mahltechniken erhalten werden. Es können ebenfalls handelsübliche Produkte eingesetzt werden, wobei die Metalloberfläche weitgehend frei von Fetten oder anderen Belegmitteln sein sollte.

Die zum Einsatz kommenden Substratteilchen sind bereits mit einer Schicht aus hochbrechendem, farblosem oder farbigem Metalloxid belegt. Geeignet sind die üblicherweise zur Beschichtung von Glanzpigmenten verwendeten Oxide wie Silicium-, Zinn-, Wismut-, Zink-, Aluminium- und Chromoxid, besonders Eisen- und Zirkondioxid und ganz besonders Titandioxid.

Metalloxidbeschichtete silikatische und metallische Pigmente sind allgemein bekannt und im Fall beschichteter Glimmerpigmente auch unter den Namen Iriodin® (E. Merck, Darmstadt), Flonac® (Kemira Oy, Pori, Finnland) und Mearlin® (Mearl Corporation, New York) im Handel. Die Herstellung der metalloxidbeschichteten Glimmerpigmente kann bekanntermaßen aus wäßriger Phase (DE-A-14 67 468, DE-A-25 22 572) oder aus der Gasphase (EP-A-45 581, DE-A-32 37 264) erfolgen, die entsprechenden Metallpigmente können ebenfalls durch Beschichtung aus der Gasphase (EP-A-33 457) oder aus alkoholischer Lösung (DE-A-35 34 477, EP-A-328 906) hergestellt werden.

Die Größe der Substratteilchen ist an sich nicht kritisch und kann auf den jeweiligen Anwendungszweck abgestimmt werden. In der Regel haben die Teilchen größte Durchmesser von etwa 1 bis 200 µm, insbesondere etwa 5 bis 100 µm und Dicken von im allgemeinen etwa 0,1 bis 5 µm, insbesondere etwa 0,5 µm.

Die Dicke der ersten Metalloxidschicht liegt in der Regel in dem für die klassischen Perlglanzpigmente bekannten Bereich von etwa 20 bis 400 nm, bevorzugt 35 bis 250 nm.

Wesentlich für die erfindungsgemäßen Glanzpigmente ist die metallsulfidhaltige Schicht, die auf die mit Metalloxid beschichteten Substratteilchen aufgebracht ist.

Zum Aufbau dieser Schicht sind vor allem die nichtselektiv absorbierenden Sulfide des Cobalts und Nickels, besonders des Eisens, Chroms und Wolframs und ganz besonders des Molybdäns geeignet. Diese Sulfide können einzeln, aber auch in Form von Gemischen, z.B. MoS₂/WS₂, vorliegen. Weiterhin sind auch Gemische mit den entsprechenden Oxiden, z.B. Gemische aus niederen Molybdänoxiden und Molybdänsulfid, oder den entsprechenden Metallen denkbar, die für die koloristischen Eigenschaften des Pigments von Vorteil sein können.

Die metallsulfidhaltige Schicht kann vorteilhaft nach dem weiter unten beschriebenen erfindungsgemäßen Verfahren über die Gasphase aufgebracht werden.

Die so erhältlichen sulfidhaltigen Schichten zeichnen sich durch gleichmäßigen, homogenen, filmartigen Aufbau aus. Je nach Schichtdicke sind sie mehr oder weniger lichtdurchlässig und tragen zur Interferenz bei.

Die sulfidischen Schichten der erfindungsgemäßen Glanzpigmente sind halbdurchlässig und haben eine Schichtdicke von 0,1 bis 50 nm, bevorzugt < 20 nm. Sie senken den Weißsockel des auftreffenden und reflektierten Lichts ab und bewirken so bei schon mit Metalloxid belegtem Substrat eine Verstärkung und je nach ihrer Schichtdicke auch eine Veränderung der Interferenzfarbe des Substrats.

Eine besonders starke Verschiebung des Farbtons ist bei MoS₂-Schichten zu beobachten. Diese wird durch den hohen Brechungsindex von n = 5,6 (λ = 500 nm) des MoS₂ hervorgerufen, der in die durch das Produkt von Brechungsindex und geometrischer Schichtdicke definierte optische Schichtdicke eingeht.

MoS₂-beschichtete Interferenzreflexionspigmente, die besonders brillant und farbstark sind, werden im blauen Farbtonbereich erhalten. Z.B. kann ein mit Titandioxid belegtes Aluminiumpigment mit zartblauer Interferenzfarbe durch MoS₂-Beschichtung in ein intensiv kornblumenblaues Pigment überführt werden.

Lichtundurchlässige sulfidische Schichten resultieren bei Schichtdicken von in der Regel > 100 nm. In diesen Fällen werden sowohl bei nur mit Sulfid als auch bei mit Oxid und Sulfid beschichtetem Substrat schwarze Glanzpigmente erhalten, die sich aufgrund der hohen Qualität der erfindungsgemäß aufgebrachten Sulfidschicht durch eine sehr glatte Oberfläche auszeichnen.

Bei fast undurchlässigen sulfidischen Schichten mit Schichtdicken von im allgemeinen etwa 20 bis 80 nm ergeben sich bei interferenzfähigem, d.h. bereits metalloxidbeschichtetem Substrat fast schwarze Glanzpigmente, die im Glanzwinkel schwach die noch durchschimmernde Interferenzfarbe des Mehrschichtsystems zeigen. Dieser Effekt kann, wenn auch in geringerem Ausmaß, bei Pigmenten beobachtet werden, die nur mit einer lichtdurchlässigen metallsulfidhaltigen Schicht belegt sind. Besonders attraktiv ist diese Art von Pigmenten für Automobillackierungen mit gedeckten Farbtönen.

Die erfindungsgemäßen Glanzpigmente zeigen also bei Belegung des interferenzfähigen Materials mit der noch durchlässigen, sulfidhaltigen Schicht in applizierter Form interessante, winkelabhängige Farb- und Helligkeitseindrücke. Je nach Betrachtungswinkel ist die Interferenzfarbe des Mehrschichtsystems oder die schwarze Farbe der nichtselektiv absorbierenden, sulfidhaltigen Schicht zu sehen.

Besteht die metallsulfidhaltige Schicht im wesentlichen aus MoS₂, dann zeigen die erfindungsgemäßen Pigmente neben den genannten Farbeffekten auch elektrische Leitfähigkeit im Bereich der Halbleiter, die sich bei Belichtung der Pigmente erhöht. Außerdem weisen diese Pigmente interessantes Reflexions- und Absorptionsverhalten im Infrarotbereich auf.

Nicht zuletzt zeichnen sich die erfindungsgemäß eingesetzten Metallsulfide und damit auch die mit ihnen beschichteten Pigmente durch ihre chemische Beständigkeit, z.B. gegenüber Säuren und Basen, und ihre Schwerlöslichkeit in Wasser aus. MoS₂ hat zudem noch ausgeprägte Hitzestabilität.

Weiterhin kann es von Vorteil sein, die erfindungsgemäßen Glanzpigmente noch mit einer Deckschicht aus farblosem oder selektiv absorbierendem Metalloxid zu belegen. In diesem Fall handelt es sich bei den sulfidhaltigen Schichten vorzugsweise um dünne Schichten von etwa 2 bis 30 nm. Diese Deckschicht kann zum Schutz der Sulfidschicht für bestimmte Anwendungszwecke dienen. Auch erhält man auf diese Weise Pigmente mit besonders gutem Deckvermögen. Werden farbige Oxide in der Deckschicht eingesetzt, so erfolgt der Farbflop von der jeweiligen Interferenzfarbe zur Eigenfarbe des Oxids, im Fall von Eisen(III)oxid also z.B. nach Rotbraun. Allgemein tragen die Oxidschichten als dünne Schichten zur Interferenz des Pigmentes bei, sie führen die Interferenzreihe an der vom Ausgangsmaterial bestimmten Stelle fort. Durch diese zusätzliche Beschichtung wird also die Farbpalette der Glanzpigmente vorteilhaft erweitert.

Für die oxidische Deckschicht können die üblichen zur Beschichtung von Interferenzpigmenten geeigneten, hochbrechenden, farblosen und farbigen, jedoch nicht schwarzen Metalloxide eingesetzt werden. Beispielsweise seien Titan-, Zirkon-, Zinn-, Chrom-, Eisen-, Aluminium-, Silicium- und Zinkoxid sowie deren Mischungen genannt. Besonders bevorzugt sind Eisen(III)oxid, Titan- und Zirkondioxid und deren Gemische.

Die Dicke der dritten Schicht ist an sich nicht kritisch, im allgemeinen beträgt sie etwa 1 bis 400 nm, insbesondere 5 bis 200 nm.

Sie kann ebenfalls vorteilhaft aus der Gasphase abgeschieden werden.

Bei dem erfindungsgemäßen Verfahren zur Herstellung von Glanzpigmenten auf Basis beschichteter, plättchenförmiger, silikatischer oder metallischer substrate, die
A) gewünschtenfalls mit einer ersten Schicht aus Metalloxid,
B) mit einer zweiten Schicht, die ein nichtselektiv absorbierendes Metallsulfid enthält, und
C) gewünschtenfalls mit einer Schicht aus Metalloxid
   belegt sind, wird die metallsulfidhaltige Schicht über die Gasphase auf das Substrat aufgebracht, das bereits eine erste Schicht aus Metalloxid aufweisen kann.

Dabei kann man nach 2 verschiedenen Verfahrensvarianten vorgehen. Bei der Variante a) wird zunächst in an sich bekannter Weise durch Gasphasenzersetzung flüchtiger Verbindungen der Metalle eine Metall- oder Metalloxidschicht auf dem Substrat abgeschieden, die anschließend durch Umsetzung mit geeigneten schwefelhaltigen Verbindungen oder Schwefeldampf in die gewünschte Sulfidschicht überführt wird. Bei der Variante b) wird eine entsprechende Metallverbindung in Gegenwart der schwefelliefernden Verbindung oder des Schwefeldampfs zersetzt und direkt als Sulfid abgeschieden. In der Regel wird die Verfahrensvariante a) vorzuziehen sein, da bei der Variante b) in der Regel größere Gasmengen von nicht umgesetzter oder gebildeter Schwefelverbindung zu befreien sind.

Das erfindungsgemäße Verfahren kann vorteilhaft in einem beheizbaren Wirbelschichtreaktor, wie er beispielsweise in der EP-A-45 851 beschrieben ist, durchgeführt werden, in dem die Substratteilchen zunächst mit einem Wirbelgas fluidisiert und auf die Zersetzungstemperatur der jeweiligen Metallverbindung von in der Regel 70 bis 350°C erhitzt werden. Die verdampften Metallverbindungen und die zur Zersetzung bzw. zur weiteren Umsetzung benötigten Gase werden dann über getrennte Düsen eingetragen.

Als flüchtige Metallverbindungen sind sowohl in Variante a) als auch in Variante b) die Carbonyle bevorzugt, also insbesondere Eisenpentacarbonyl, Chrom-, Molybdän- und Wolframhexacarbonyl, daneben Nickeltetracarbonyl und dimeres Cobalttetracarbonyl.

Sollen bei der Variante a) metallische Schichten abgeschieden werden, dann werden die Carbonyle, wie in der älteren deutschen Patentanmeldung P 4141069.6 beschrieben, in Gegenwart eines inerten Gases wie Stickstoff oder Argon zersetzt.

Sollen zunächst Metalloxidschichten aufgebracht werden, so sind neben den üblichen Oxiden wie Fe₂O₃, Cr₂O₃, MoO₃, WO₃, NiO und CoO auch niedere Oxide wie Fe₃O₄ und vor allem Molybdän- und Wolframsuboxide geeignet.

Die Abscheidung der üblichen Metalloxide kann, wie beispielsweise in der EP-A-45 851 beschrieben, in Gegenwart von Sauerstoff (bzw. Luft) in An- oder Abwesenheit von Wasserdampf erfolgen. Weiterhin können auch zunächst Metallschichten (z.B. Mo) abgeschieden und dann beispielsweise mit Luft bei Temperaturen von in der Regel 300 bis 400°C zu Oxidschichten (z.B. MoO₃) oxidiert werden.

Zur Erzeugung einer Fe₃O₄-Schicht wird das Eisencarbonyl in Abwesenheit von Sauerstoff mit Wasserdampf hydrolysiert (s. ebenfalls P 4141069.6).

Molybdän- bzw. Wolframsuboxidschichten können, wie in der älteren deutschen Anmeldung P 4221010.0 beschrieben, durch Zersetzung der Carbonyle mit Sauerstoff bzw. Luft/Stickstoff-Gemischen in An- oder Abwesenheit von Wasserdampf erhalten werden. Dazu sollte der Sauerstoff in der Regel 0,5 bis 10 Vol.-%, vorzugsweise 0,8 bis 6 Vol.-%, und der Wasserdampf etwa 0 bis 2 Vol.-% der Gesamtgasmenge im Reaktor ausmachen. Die erforderliche Reaktionstemperatur beträgt im allgemeinen 150 bis 250°C, bevorzugt 200 bis 220°C.

Wie für die Gasphasenzersetzung (chemical vapour deposition, CVD) im Wirbelschichtreaktor allgemein üblich, werden die Metallcarbonyle zweckmäßigerweise in einer dem Reaktor vorgeschalteten Verdampfervorlage verdampft, mit einem inerten Trägergas, bevorzugt Stickstoff, in den Reaktor transportiert und dort durch die dem Wirbelgas zugemischten Reaktionsgase (Sauerstoff bzw. Luft und/oder Wasserdampf) zersetzt.

Um das Substrat gleichmäßig und vollständig umhüllende, homogene Schichten zu erhalten, sollte die Gasmenge an Metallcarbonyl im allgemeinen nicht mehr als 5 Vol.-%, vorzugsweise nicht mehr als 2 Vol.-% der Gesamtgasmenge im Reaktor betragen.

Die bei der Variante a) erhaltenen Metall- bzw. Metalloxidschichten können ohne Zwischenisolierung in demselben Reaktor unter Verwendung eines inerten Wirbelgases (bevorzugt Stickstoff), dem die flüchtige schwefelhaltige Verbindung oder der Schwefeldampf zugesetzt ist, in die gewünschten sulfidischen Schichten überführt werden. Diese Umsetzung kann jedoch auch nach Zwischenisolierung des beschichteten Substrats in anderen Aggregaten wie gasdichten Drehrohröfen oder Kammeröfen vorgenommen werden.

Analog wird bei der Variante b) das eingesetzte Metallcarbonyl in Gegenwart der schwefelliefernden Verbindung oder des Schwefeldampfs und eines inerten Wirbelgases zersetzt. Die Konzentration der gasförmigen schwefelhaltigen Verbindung bzw. des Schwefeldampfs sollte dabei nicht mehr als 5 Vol.-%, vorzugsweise 2 bis 4 Vol.-% betragen.

Als schwefelhaltige Verbindungen eignet sich bei beiden Varianten neben C₁-C₅-Thioalkoholen und C₂-C₄-Thioethern, wie Methyl-, Ethyl-, Propyl-, Butyl-, Pentylmercaptan, Dimethylsulfid und Diethylsulfid, Schwefelkohlenstoff und elementarem Schwefel vor allem Schwefelwasserstoff.

Die Reaktionstemperaturen betragen in der Regel 200 bis 500°C, bevorzugt 300 bis 400°C.

Nach abgeschlossener Belegung mit der sulfidhaltigen Schicht empfiehlt es sich, den Reaktor zur Entfernung von Restmengen an Schwefelwasserstoff bzw. den anderen Schwefelverbindungen noch etwa 1 bis 2 h mit Stickstoff zu spülen. Überschüssiger Schwefelwasserstoff oder bei der Umsetzung gebildeter Schwefel oder gebildetes Schwefeldioxid kann dann in einfacher Weise durch Wäsche mit Natronlauge aus dem Abgas entfernt werden.

Falls gewünscht, kann auf die mit der Sulfidschicht belegten Substratteilchen noch in üblicher Weise eine oxidische Deckschicht aufgebracht werden. Dies kann zur Verbesserung von Licht- oder Wetterechtheit von Vorteil sein.

Als Ausgangsverbindungen können hier neben den Carbonylen auch die Halogenide, vor allem Chloride, sowohl aromatische Alkoholate wie Phenolate und Benzylakoholate als auch aliphatische, vor allem C₁-C₄-Alkanolate wie n-, iso-, tert.-Butanole, bevorzugt Methanolate und Ethanolate und besonders bevorzugt n- und iso-Propanolate, eingesetzt werden. Die Halogenide und Alkoholate werden dabei, wie in der älteren deutschen Patentanmeldung P 4217511.9 beschrieben, durch Wasserdampf hydrolysiert, wobei im Fall der Halogenide Sauerstoff (bzw. Luft) anwesend sein kann. Beispiele für bevorzugte Verbindungen sind Eisenpentacarbonyl, Chromhexacarbonyl, Aluminiumchlorid, Silicium-, Zinn-, Titan- und Zirkon-tetrachlorid und Titan- und Zirkon-n- und -iso-propanolat.

Die Zersetzung dieser Metallverbindungen wird zweckmäßigerweise bei Temperaturen < 250°C, insbesondere von 100 bis 200°C, vorgenommen. Welche Metallverbindung bevorzugt eingesetzt wird, hängt von der aufgebrachten Sulfidschicht ab. Sind die sulfidhaltigen Schichten unter diesen Bedingungen oxidationsempfindlich, wird z.B. vorzugsweise unter Verwendung der Alkoholate beschichtet.

Das erfindungsgemäße Verfahren ermöglicht den Einbau sulfidhaltiger, hochbrechender, stark absorbierender Schichten in mehrphasige Pigmente und damit die reproduzierbare Herstellung qualitativ hochwertiger Glanzpigmente, die sich durch homogene, gleichmäßige, die Substratteilchen filmartig umhüllende Schichten besonders auszeichnen.

Die erfindungsgemäßen Glanzpigmente eignen sich vorteilhaft für viele Zwecke, z.B. zur Einfärbung von Lacken, Druckfarben, Kunststoffen, Gläsern, keramischen Produkten und Zubereitungen der dekorativen Kosmetik. Der stark winkelabhängige Farbeindruck beim Abkippen der applizierten erfindungsgemäßen interferenzfähigen Pigmente ermöglicht die Herstellung von Sicherheits- und Effektfarben, vor allem für den Wertpapierdruck und für fälschungssichere Dokumente. In diesem Zusammenhang ist auch die hohe Säure- und Laugenbeständigkeit von MoS₂ von besonderem Interesse. Aufgrund ihres hohen Deckvermögens und ihre attraktiven Koloristik sind die molybdän- oder eisensulfidbeschichteten Pigmente insbesondere für die Automobillackierung interessant, da bei ihrer Anwendung auf den Einsatz sonst erforderlicher Grundierungsmittel und weiterer Farbmittel verzichtet werden kann. Zusätzlich erleichtern die günstigen Oberflächeneigenschaften MoS₂-beschichteter Glanzpigmente (MoS₂ selbst wird vielfach als Gleitmittel verwendet) ihre Einarbeitung in die Anwendungsmedien.

### Beispiele

### Herstellung von Glanzpigmenten nach dem erfindungsgemäßen Verfahren

Die in den Beispielen beschriebenen Beschichtungen von Glimmer- bzw. TiO₂-beschichteten Glimmerpigmenten sowie Aluminiumpigmenten mit Metall oder Metalloxid wurden jeweils in einem von außen beheizbaren Wirbelschichtreaktor aus Glas mit einem Durchmesser von 8 cm und einer Höhe von 80 cm mit Glasfrittenboden und oben eingehängten, mit einem Stickstoff-Jet abreinigenden Filterstrümpfen und zwei seitlich oberhalb des Frittenbodens eingebrachten Düsen zur Gaseinleitung durchgeführt.

Die Überführung der Metall- bzw. Metalloxidschicht in die gewünschte Sulfidschicht erfolgte in einer Apparatur, die im wesentlichen aus einem über einen Motor drehbaren und durch einen zweischaligen Klappofen beheizbaren Quarzrundkolben mit Gaszu- und -ableitung in der Drehachse ("Drehkugelofen") (Beispiele 1 bis 7) oder im Wirbelschichtreaktor (Beispiele 8 und 9).

### A) Herstellung von metallsulfidbeschichteten Glimmerpigmenten

### Beispiele 1 bis 4

200 g des in Tabelle 1 angegebenen Glimmerpigments (Rohglimmer bzw. TiO₂-beschichteter Glimmer) wurden im Wirbelschichtreaktor unter Verwirbelung mit insgesamt 800 l/h Stickstoff auf 220°C erhitzt. Dabei wurde die Hälfte der Wirbelgase über eine auf 70°C erwärmte Vorlage mit Molybdänhexacarbonyl geleitet. Innerhalb von 8 h wurden so x g Mo(CO)₆ zugeführt, das sich als hydrolyseempfindlicher Molybdänfilm auf dem Substrat abschied.

50 g des schwarzen molybdänbeschichteten Pigments wurden im Drehkugelofen unter Durchleiten von Luft auf 300°C erhitzt, wobei Oxidation des Molybdäns zum farblosen Molybdäntrioxid erfolgte. Nach zweistündiger Inertisierung mit Stickstoff wurden bei 400 bis 450°C 6 l Schwefelwasserstoff (2 l/h) durchgeleitet. Dabei wurde MoO₃ unter zusätzlicher Bildung von Wasserdampf und Schwefel in das schwarze Molybdänsulfid MoS₂ überführt. Der entstandene Schwefel süblimierte ab und wurde in 10 gew.-%iger Natronlauge als Kolloid abgeschieden.

Nach beendeter Reaktion wurde das Produkt innerhalb von 3 h unter Inertisierung auf Raumtemperatur abgekühlt.

Die so erhaltenen molybdänsulfidbeschichteten Pigmente zeigten starken Glanz, ausgezeichnetes Deckvermögen und keine Empfindlichkeit gegen Wasser.

Weitere Einzelheiten zu diesen Versuchen sowie deren Ergebnisse sind in der folgenden Tabelle zusammengestellt.

**Tabelle**

| Bsp. | Glimmerpigment | x g Mo(CO)₆ | Mo-Gehalt [Gew.-%] | S-Gehalt [Gew.-%] | Farbton bzw. Farbflop |
|---|---|---|---|---|---|
| 1 | naßgemahlener Muskovit, Teilchengröße < 100 µm | 43,6 | 3,9 | 2,0 | grauschwarz |
| 2 | Iriodin® 9205 Rutil Brillantgelb WR (Merck) | 20,2 | 3,2 | 2,2 | kupfer |
| 3 | Iriodin 9225 Rutil Perlblau WR | 40,8 | 5,7 | 4,1 | schwarz → grünstichig blau |
| 4 | Iriodin 9215 Rutil Perlrot WR | 50,3 | 7,8 | 6,2 | schwarz → schwachgrün |

### Beispiel 5

200 g des TiO₂-beschichteten Glimmerpigments Iriodin 9235 Rutil Perlgrün WR wurden analog Beispiel 1, jedoch unter zusätzlicher Einleitung von 50 l/h durch eine auf 50°C temperierte Wasservorlage geleitete Luft und unter Zuführung von 76,9 g Mo(CO)₆ mit blauem Molybdänsuboxid belegt. Das erhaltene Pigment zeigte einen Farbflop von blau nach grün.

50 g dieses Pigments wurden analog Beispiel 1 mit Schwefelwasserstoff zu schwarzem molybdänsulfidbeschichtetem Pigment umgesetzt (9,0 Gew.-% Mo, 4,7 Gew.-% S).

Appliziert in Lack, war bei dem schwarzen Pigment unter bestimmtem Betrachtungswinkel schwach die grüne Interferenzfarbe zu erkennen.

Das Pigment veränderte sich weder in kochendem Wasser noch in 80°C heißer Salzsäure (pH-Wert 1).

### Beispiel 6

In einem etwas größer ausgelegten, ansonsten baugleichen Wirbelschichtreaktor wurden 800 g des TiO₂-beschichteten Glimmerpigments Iriodin 9103 Sterling Silber WR unter Verwirbelung mit insgesamt 2000 l/h Stickstoff auf 180°C erhitzt. Dabei wurde ein Fünftel der Wirbelgase über eine auf 35°C temperierte Vorlage mit Eisenpentacarbonyl geleitet. Insgesamt wurden so 870 g Fe(CO)₅ zugeführt. Gleichzeitig wurden zur Oxidation (Bildung von Fe₂O₃) 600 l/h über eine auf 50°C temperierte Wasservorlage geleitete Luft eingeblasen.

50 g des braunroten, Fe₂O₃-beschichteten Pigments wurden analog Beispiel 1 mit Schwefelwasserstoff zu schwarzem, eisensulfidbeschichtetem Pigment (17,9 Gew.-% Fe, 11,4 Gew.-% S) umgesetzt, das, in Lack appliziert, unter bestimmtem Betrachtungswinkel schwachen Goldglanz zeigte.

### Beispiel 7

200 g des TiO₂-beschichteten Glimmerpigments Iriodin 9225 Rutil Perlblau WR wurden unter Verwirbelung mit insgesamt 800 l/h Stickstoff auf 220°C erhitzt. Dabei wurde die Hälfte der Wirbelgase über eine auf 35°C temperierte Vorlage mit 60,0 g Eisenpentacarbonyl geleitet. Zur oberflächigen Passivierung der auf den Substratteilchen abgeschiedenen Eisenschicht wurden nach Abkühlen auf 100°C in 30 min 25 l Luft zugeführt.

50 g dieses Pigments wurden analog Beispiel 1 mit Schwefelwasserstoff zu tiefblauem eisensulfidbeschichtetem Pigment (7,4 Gew.-% Fe, 7,1 Gew.-% S) umgesetzt.

### B) Herstellung von metallsulfidbeschichteten Aluminiumpigmenten

### Beispiel 8

Eine Mischung aus 100 g Aluminiumpulver (mittlerer Teilchendurchmesser 20 µm, BET-Oberfläche 4,5 m²/g) und 100 g gröberem Aluminiumpulver (mittlerer Teilchendurchmesser 60 µm, BET-Oberfläche 1,5 m²/g) wurde unter Verwirbelung mit insgesamt 800 l/h Stickstoff auf 220°C erhitzt. Dabei wurde die Hälfte der Wirbelgase über eine auf 70°C erwärmte Vorlage mit Molybdänhexacarbonyl geleitet. Innerhalb von 10 h wurden so 81,7 g Mo(CO)₆ zugeführt, das durch gleichzeitiges Zudosieren von 200 l/h Luft zu Molybdäntrioxid oxidiert wurde und sich als Oxidfilm auf dem Substrat abschied.

Anschließend wurde zunächst die Luftzufuhr und nach weiteren 30 min auch die Stickstoffzufuhr gestoppt. Nach Temperaturerhöhung auf 300°C wurde über 10 h ein Gemisch aus 1 l/h Schwefelwasserstoff und 4 l/h Stickstoff eingeleitet.

Es wurde ein schwarzes Pigment mit schwach goldener Interferenzfarbe (19,0 Gew.-% Mo, 17,8 Gew.-% S) erhalten.

### Beispiel 9

200 g der Aluminiummischung aus Beispiel 8 wurden unter Verwirbelung mit insgesamt 800 l/h Stickstoff auf 180°C erhitzt. Dabei wurde eine Hälfte der Wirbelgase über eine auf 35°C temperierte Vorlage mit Titantetrachlorid und die andere Hälfte über eine auf 35°C temperierte Wasservorlage geleitet. Innerhalb von 10 h wurden so 65,0 g TiCl₄ zugeführt, das sich als Titandioxidfilm auf den Aluminiumteilchen abschied.

Nach beendeter TiO₂-Abscheidung wurde die Reaktortemperatur auf 220°C erhöht. Dann wurden 400 l/h Stickstoff über eine auf 80°C erwärmte Vorlage mit Molybdänhexacarbonyl geleitet. In 10 h wurden so 81,7 g Mo(CO)₆ zugeführt, das durch gleichzeitiges Zudosieren von 50 l/h Luft zu Molybdänsuboxid oxidiert wurde und sich filmartig auf dem Substrat abschied.

Anschließend wurde das molybdänbeschichtete, blaue Pigment analog Beispiel 8 mit Schwefelwasserstoff zu einem intensiv kornblumenblauen Metallicpigment (8,9 Gew.-% Mo, 1,9 Gew.-% S) umgesetzt.

## Patentansprüche

1. Glanzpigmente auf Basis beschichteter, plättchenförmiger, silikatischer oder metallischer Substrate, die mit
A) einer ersten Schicht aus Metalloxid,
B) einer zweiten Schicht, die ein nichtselektiv absorbierendes Metallsulfid enthält und eine Schichtdicke von 0,1 bis 50 nm aufweist, und
C) gewünschtenfalls einer dritten Schicht aus Metalloxid
belegt sind.

2. Glanzpigmente nach Anspruch 1, bei denen die metallsulfidhaltige Schicht aus einem Metallsulfid oder einem Gemisch verschiedener Metallsulfide oder einem Gemisch aus Metallsulfid und dem jeweiligen Metalloxid oder einem Gemisch aus Metallsulfid und dem jeweiligen Metall besteht.

3. Glanzpigmente nach Anspruch 1 oder 2, bei denen die metallsulfidhaltige Schicht Chrom-, Molybdän-, Wolfram-, Eisen-, Cobalt- und/oder Nickelsulfid enthält.

4. Glanzpigmente nach den Ansprüchen 1 bis 3, die mit einer Schicht aus Titan-, Zirkon-, Zinn-, Eisen-, Chrom-, Vanadin-, Cobalt- und/oder Nickeloxid belegt sind.

5. Verfahren zur Herstellung von Glanzpigmenten auf Basis beschichteter, plättchenförmiger, silikatischer oder metallischer Substrate, die
A) gewünschtenfalls mit einer ersten Schicht aus Metalloxid,
B) mit einer zweiten Schicht, die ein nichtselektiv absorbierendes Metallsulfid enthält, und
C) gewünschtenfalls mit einer dritten Schicht aus Metalloxid
belegt sind,
dadurch gekennzeichnet, daß man das gewünschtenfalls bereits mit einer Metalloxidschicht belegte Substrat
a) zunächst in bekannter Weise durch Gasphasenzersetzung flüchtiger Metallverbindungen in Gegenwart eines Inertgases oder von Sauerstoff und/oder Wasserdampf mit der entsprechenden Metall- oder Metalloxidschicht belegt und diese dann durch Umsetzung mit einer flüchtigen, schwefelhaltigen Verbindung oder Schwefeldampf in die gewünschte metallsulfidhaltige Schicht überführt oder
b) durch Gasphasenzersetzung flüchtiger Metallverbindungen in Gegenwart einer flüchtigen, schwefelhaltigen Verbindung oder Schwefeldampf direkt mit der metallsulfidhaltigen Schicht belegt
sowie das so beschichtete Substrat gewünschtenfalls anschließend in üblicher Weise mit einer weiteren Metalloxidschicht belegt.

6. Verfahren nach Anspruch 5, dadurch gekennzeichnet, daß man als flüchtige, schwefelhaltige Verbindung Schwefelwasserstoff, Schwefelkohlenstoff, C₁-C₅-Thioalkohole oder C₂-C₄-Thioether einsetzt.

7. Verfahren nach Anspruch 5 oder 6, dadurch gekennzeichnet, daß man als flüchtige Metallverbindungen die Carbonyle einsetzt.

8. Verwendung der Glanzpigmente gemäß den Ansprüchen 1 bis 4 zur Einfärbung von Lacken, Druckfarben, Kunststoffen, Gläsern, keramischen Produkten und Zubereitungen der dekorativen Kosmetik.

## Claims

1. Luster pigments based on coated, platelet-shaped, silicatic or metallic substrates wherein the coating comprises
A) a first layer comprising a metal oxide,
B) a second layer from 0.1 to 50 mm in thickness comprising a nonselectively absorbing metal sulfide, and
C) if desired a third layer comprising a metal oxide.

2. Luster pigments as claimed in claim 1 wherein the metal sulfide layer comprises a metal sulfide or a mixture of various metal sulfides or a mixture of metal sulfide and the respective metal oxide or a mixture of a metal sulfide and the respective metal.

3. Luster pigments as claimed in claim 1 or 2 wherein the metal sulfide layer comprises chromium sulfide, molybdenum sulfide, tungsten sulfide, iron sulfide, cobalt sulfide or nickel sulfide.

4. Luster pigments as claimed in claims 1 to 3 wherein the coating comprises titanium oxide, zirconium oxide, tin oxide, iron oxide, chromium oxide, vanadium oxide, cobalt oxide or nickel oxide.

5. A process for preparing luster pigments based on coated, platelet-shaped, silicatic or metallic substrates wherein the coating comprises
A) if desired, a first layer comprising a metal oxide,
B) a second layer comprising a nonselectively absorbing metal sulfide, and
C) if desired, a third layer comprising a metal oxide,
characterized in that it comprises coating the substrate, which may if desired have already been coated with a metal oxide, with
a) a metal or metal oxide layer produced in a conventional manner by gas phase decomposition of volatile metal compounds in the presence of an inert gas or of oxygen and/or water vapor and thereafter converted by reaction with a volatile sulfur-containing compound or sulfur vapor into the desired metal sulfide coating, or
b) directly with the metal sulfide layer by gas phase decomposition of volatile metal compounds in the presence of a volatile sulfur-containing compound or sulfur vapor,
and if desired then coating the thusly coated substrate with a further metal oxide layer in a conventional manner.

6. A process as claimed in claim 5, characterized in that the volatile sulfur-containing compound used is hydrogen sulfide, carbon disulfide, a C₁-C₅-thioalcohol or a C₂-C₄-thioether.

7. A process as claimed in claim 5 or 6, characterized in that the volatile metal compound used is a carbonyl.

8. The use of the luster pigments of claims 1 to 4 for coloring paints, printing inks, plastics, glasses, ceramic products and decorative cosmetic preparations.

## Revendications

1. Pigments brillants à base de substrats en paillettes enduits, en silicate ou en métal, qui sont revêtus
A) d'une première couche d'oxyde métallique,
B) d'une deuxième couche qui contient un sulfure métallique à absorption non sélective et qui présente une épaisseur de couche de 0,1-50 nm, et
C) éventuellement d'une troisième couche d'oxyde métallique.

2. Pigments brillants selon la revendication 1, dans lesquels la couche contenant un sulfure métallique est faite d'un sulfure métallique ou d'un mélange de différents sulfures métalliques, ou d'un mélange de sulfure métallique et de l'oxyde métallique correspondant ou d'un mélange de sulfure métallique et du métal correspondant.

3. Pigments brillants selon la revendication 1 ou 2, dans lesquels la couche contenant un sulfure métallique contient du sulfure de chrome, de molybdène, de tungstène, de fer, de cobalt et/ou de nickel.

4. Pigments brillants selon l'une quelconque des revendications 1 à 3, qui sont revêtus d'une couche d'oxyde de titane, de zirconium, d'étain, de fer, de chrome, de vanadium, de cobalt et/ou de nickel.

5. Procédé de préparation de pigments brillants à base de substrats en paillettes enduits, en silicate ou en métal, qui sont revêtus
A) éventuellement avec une première couche d'oxyde métallique,
B) avec une deuxième couche qui contient un sulfure métallique à absorption non sélective, et
C) éventuellement avec une troisième couche d'oxyde métallique, caractérisé en ce que le substrat, au besoin déjà revêtu d'une couche d'oxyde métallique,
a) est d'abord revêtu de façon connue, par décomposition en phase gazeuse de composés métalliques volatils, en présence d'un gaz inerte ou d'oxygène et/ou de vapeur d'eau, de la couche de métal ou d'oxyde métallique correspondante, puis cette couche est transformée, par réaction avec un composé volatil contenant du soufre ou avec de la vapeur de soufre, en la couche désirée contenant un sulfure métallique, ou bien
b) il est revêtu directement de la couche contenant un sulfure métallique, par décomposition en phase gazeuse de composés métalliques volatils en présence d'un composé volatil contenant du soufre ou de vapeur de soufre,
et le substrat ainsi enduit est ensuite revêtu au besoin, de la manière habituelle, d'une autre couche d'oxyde métallique.

6. Procédé selon la revendication 5, caractérisé en ce que l'on utilise, comme composé volatil contenant du soufre, de l'acide sulfhydrique, du sulfure de carbone, des thioalcools en C₁-C₅ ou des thioéthers en C₂-C₄.

7. Procédé selon la revendication 5 ou 6, caractérisé en ce que l'on utilise les carbonyle comme composés métalliques volatils.

8. Utilisation des pigments brillants selon l'une quelconque des revendications 1 à 4 pour la coloration de laques, d'encres d'imprimerie, de matières plastiques, de verres, de produits céramiques et de préparations de la cosmétique décorative.
